(19) Europäisches Patentamt
European Patent Office
Office européen des brevets

(11) EP 0 537 519 B1

# (12) EUROPÄISCHE PATENTSCHRIFT

(45) Veröffentlichungstag und Bekanntmachung des Hinweises auf die Patenterteilung:
**04.06.1997 Patentblatt 1997/23**

(51) Int Cl.6: **C07D 317/46**, C07D 317/20

(21) Anmeldenummer: **92116318.4**

(22) Anmeldetag: **24.09.1992**

(54) **Verfahren zur Herstellung von fluorsubtituierten Aminobenzodioxolen und -benzodioxanen und neue Zwischenprodukte**

Method for the preparation of fluoro substituted aminobenzodioxoles and -benzodioxanes and intermediates

Procédé pour la préparation d'aminobenzodioxoles et -benzodioxanes fluoro substitués et intermédiaires

(84) Benannte Vertragsstaaten:
**BE CH DE ES FR GB IT LI NL**

(30) Priorität: **07.10.1991 DE 4133156**

(43) Veröffentlichungstag der Anmeldung:
**21.04.1993 Patentblatt 1993/16**

(73) Patentinhaber: **BAYER AG**
**51368 Leverkusen (DE)**

(72) Erfinder:
- **Andres, Peter, Dr.**
  **W-5653 Leichlingen 2 (DE)**
- **Marhold, Albrecht, Dr.**
  **W-5090 Leverkusen 1 (DE)**

(56) Entgegenhaltungen:
**EP-A- 206 999**
**EP-A- 637 518**
**EP-A- 0 011 179**
**EP-A- 0 198 797**
**EP-A- 0 291 799**
**DE-A- 3 642 256**
**GB-A- 2 062 888**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein neues Verfahren zur Herstellung von fluorsubstituierten Aminobenzodioxolen und -benzodioxanen, die als wertvolle Ausgangsverbindungen für die Synthese hoch aktiver Verbindungen zur Bekämpfung z.B. von Schädlingen gebraucht werden, und weiterhin neue Zwischenprodukte.

Es ist bekannt, daß fluorsubstituierte Aminobenzodioxole erhältlich sind, wenn man 4-Carboxy-1,3-benzodioxol (vgl. W.H. Perkin und V.M. Trikojus, J. Chem. Soc. 1926, 2925) mit Phosphorpentachlorid zum 2,2-Dichlor-1,3-benzodioxol-4-carbonylchlorid umsetzt, dieses mit Antimontrifluorid zur entsprechenden Trifluorverbindung umsetzt, woraus mit Ammoniak das 4-Carboxamid hergestellt wird, das in einer vierten und letzten Stufe durch Hofmann Abbau, d.h. durch Umsetzung mit Brom und Alkalihydroxid zum gewünschten 2,2-Difluor-4-amino-1,3-benzodioxol umgesetzt wird (vgl. EP-198.797).

Nachteile dieses bekannten Verfahrens sind die Vierstufenreaktion. Nachteilig sind außerdem die teuren Reagenzien wie Phosphorpentachlorid und Antimontrifluorid, die außerdem Probleme bei der Entsorgung machen, d.h. die Abfall- und Nebenprodukte davon. Außerdem ist das einzusetzende Ausgangsprodukt - das 4-Carboxy-1,3-benzodioxol - ebenfalls über eine vielstufige Synthese erhältlich. Insgesamt kann man feststellen, daß der Zugang der gewünschten fluorsubstituierten Aminoverbindungen über das Amid sehr aufwendig ist und für eine großtechnische Durchführung nicht geeignet ist.

Es wurde nun gefunden, daß man die fluorsubstituierten Amino-benzodioxole und -benzodioxane der Formel

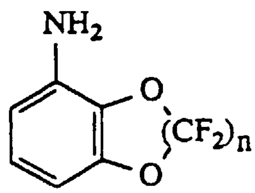

(I)

in welcher

n für eine ganze Zahl 1 oder 2 steht, erhält,

wenn man fluorsubstituierte Benzodioxole oder Benzodioxane der Formel

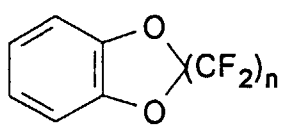

(II)

in welcher

n die oben angegebene Bedeutung hat,

in einer ersten Stufe mit elementarem Halogen in Gegenwart von Halogenierungskatalysatoren und gegebenenfalls in Gegenwart von Lösungs- oder Verdünnungsmitteln umsetzt und

in einer zweiten Stufe die so erhältichen halogensubstituierten Benzodioxole und Benzodioxane der Formel

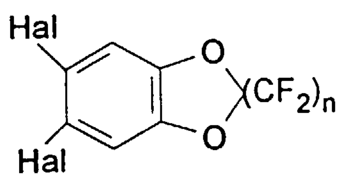

(III)

in welcher

n die obengenannte Bedeutung hat und
Hal für Halogen, vorzugsweise für Chlor oder Brom steht,

mit Nitriersäure ohne zusätzliche Lösungsmittel umsetzt und

in einer dritten Stufe die so erhältlichen Nitroverbindungen der Formel

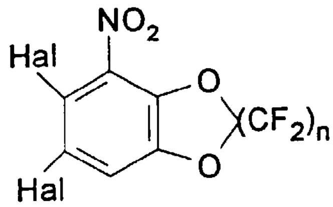

(IV)

in welcher

Hal und n die angegebenen Bedeutungen haben,

in Gegenwart von Palladiumkatalysatoren in Gegenwart eines Lösungsmittels und gegebenenfalls in Gegenwart einer Base mit Wasserstoff hydriert.

Es ist als ausgesprochen überraschend zu bezeichnen, daß gemäß der erfindungsgemäßen Umsetzung die gewünschten Produkte in hoher Reinheit und guten Ausbeuten erhalten werden. Vor allem ist es überraschend, daß die letzte Stufe die katalytische Hydrierung sowohl die Nitrogruppe reduziert als auch die Halogenatome im ankondensierten Benzoring abspaltet ohne daß Nebenreaktionen beobachtet wurden.

Das erfindungsgemäße Verfahren besitzt gegenüber dem vorbekannten Verfahren eine Reihe von Vorteilen:

Erstens wird eine Reaktionsstufe eingespart. Zweitens ist das verwendete Ausgangsmaterial das 2,2-Difluor-1,3-benzodioxol auch in technischen Mengen käuflich zu erwerben. Weiterhin wird bei der Halogenierung elementares Halogen eingesetzt, das nur gasförmig entweichende Nebenprodukte liefert und somit das Abwasser nicht belastet, während das beim bekannten Verfahren einzusetzende Phosphorpentachlorid als Reaktionsprodukt Phosphoroxichlorid bildet, das zur Abtrennung einen besonderen Aufwand erfordert. Außerdem wird der Einsatz des teuren Antimontrifluorids vermieden, dessen Nebenprodukte das Abwasser belasten und das selbst schwer rückgewinnbar ist. Der erfindungsgemäß in der dritten Stufe einzusetzende Palladium/Kohle Katalysator ist allgemein durch Abfiltrieren zu entsorgen und rückgewinnbar.

Verwendet man beispielsweise 2,2-Difluor-1,3-benzodioxol, elementares Chlor in Gegenwart von Eisen, anschließend als Nitriermittel ein Salpeter-/Schwefelsäuregemisch und in der letzten Stufe Wasserstoff und als Katalysator Palladium-Kohle, so läßt sich der Reaktionsablauf des erfindungsgemäßen Verfahrens durch das folgende Formelschema darstellen:

1.

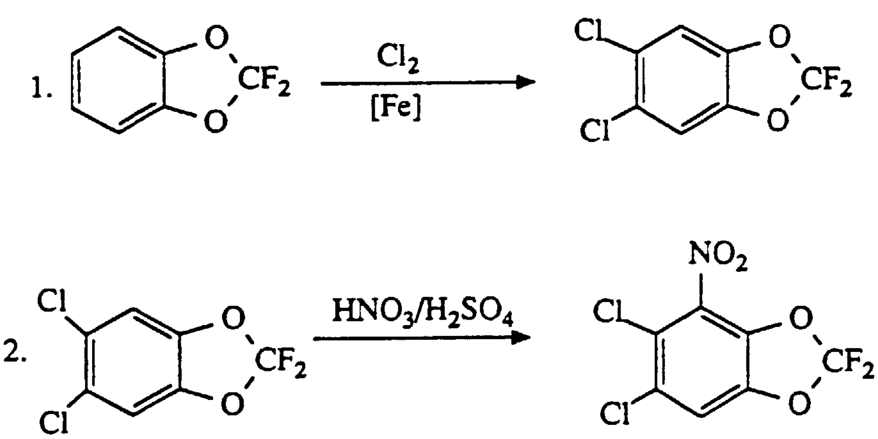


2.

3.

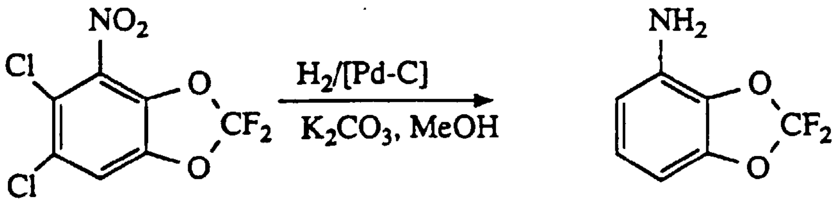


Die zur Durchführung des erfindungsgemäßen Verfahrens als Ausgangsstoffe benötigten fluorsubstituierten Benzodioxole bzw. Benzodioxane der Formel (II) sind bekannt und wenigstens das Dioxol käuflich zu erwerben, aber alle Ausgangsverbindungen sind nach Analogieverfahren herstellbar (DE 3.315.147 ≙ C.A. Vol. 102; 78862b, EP-A 0 011 179 und DE 3.642.256). Das elementare Halogen, die üblichen Halogenierungskatalysatoren ebenso wie die Nitriersäure sind gängige Chemikalien.

Die halogensubstituierten Benzodioxole bzw. Benzodioxane der Formel (III) sind neu, die Halogenierung ist allerdings eine altbekannte Methode (vgl. DE 2.819.788). Sie sind auch Gegenstand der Erfindung.

Die weiterhin auftretenden Zwischenprodukte der Formel (IV) sind auch neu und ebenfalls Gegenstand der Erfindung. Man erhält diese neuen Verbindungen aus den Verbindungen der Formel (III) durch Nitrierung. Vergleiche dazu auch die Herstellungsbeispiele.

Als Halogenierungsmittel in der Stufe 1 von Verbindungen der Formel (II) zu denen der Formel (III) werden elementare Halogene eingesetzt, vorzugsweise elementares Chlor oder Brom.

Als Katalysatoren in der Stufe 1 werden übliche Lewis-Säuren als Halogenierungskatalysatoren eingesetzt, vorzugsweise Eisen und Eisen(III)chlorid, Eisen(III)bromid und Aluminium(III)chlorid.

Die 1. Stufe wird vorzugsweise ohne Lösungsmittel durchgeführt. Generell kommen aber Lösungsmittel in Frage, z.B. chlorierte Kohlenwasserstoffe, wie Chloroform, Dichlormethan, Tetrachlorkohlenstoff.

Die Temperaturen können in einem größeren Bereich variiert werden. Im allgemeinen arbeitet man bei Temperaturen zwischen -80°C und 160°C, vorzugsweise bei Temperaturen zwischen -30°C und 80°C, besonders bevorzugt bei Temperaturen zwischen -10°C und 50°C.

Das Verhältnis von eingesetztem Halogen zu Ausgangsmaterial der Formel (II) ist vorzugsweise 2:1.

Die Reaktionstemperaturen können in einem größeren Bereich variiert werden. Im allgemeinen arbeitet man bei Temperaturen zwischen -50°C und 160°C, bevorzugt bei Temperaturen zwischen -20°C und 80°C, besonders bei Temperaturen zwischen -10°C und 40°C.

Die Nitriersäure setzt man zum Ausgangsmaterial im allgemeinen im Verhältnis 2:1 bis 10:1, bevorzugt 4:1 ein.

Die Stufe 3, die katalytische Hydrierung wird in einem Lösungsmittel durchgeführt, z.B. in Alkoholen, wie Methanol oder Ethern, wie Tetrahydrofuran.

Als Basenzusätze werden bevorzugt Alkalicarbonate, wie Kaliumcarbonat oder tertiäre Amine, wie Triethylamin verwendet, wobei das Verhältnis von Base zu Ausgangsmaterial im allgemeinen 2:1 beträgt.

Die Reaktionstemperaturen bei Stufe 3 können in einem größeren Bereich variiert werden. Man arbeitet im allgemeinen bei Temperaturen zwischen 20°C und 180°C, bevorzugt bei Temperaturen zwischen 30°C und 140°C, besonders bevorzugt bei Temperaturen zwischen 30°C und 100°C.

Der Wasserstoffdruck liegt im allgemeinen im Bereich zwischen 1 und 200 bar, bevorzugt im Bereich zwischen 50 und 150 bar, besonders bevorzugt im Bereich zwischen 70 und 120 bar.

Die mit Hilfe des erfindungsgemäßen Verfahrens erhältlichen fluorsubstituierten Amino-benzodioxole und -benzodioxane können eingesetzt werden als Ausgangsverbindungen für hochaktive Biozide, z.B. zur Bekämpfung von Schädlingen, vorzugsweise pflanzenschädigenden Pilzen, Bakterien, Insekten und Akariden. Es handelt sich dabei z. B. um das 2,2-Difluor-4-(2,4-dinitro-6-trifluoranilino)-1,3-benzodioxol (vgl. EP 198.797).

Z.B. ist der genannte Wirkstoff als Mikrobizid gegen die den folgenden Klassen angehörenden phytopathogenen Pilze wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); sowie gegen die der Klasse Phycomycetes angehörenden Oomycetes (z.B. Plasmopara viticola); insbesondere gegen die Klasse der Ascomyceten (z.B. Venturia, Podosphaera, Erysiphe, Monilinia, Uncinula). Überdies wirkt die Verbindung systemisch. Sie kann ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Als Insektizid kann die Verbindung auch zur Bekämpfung von Insekten der Ordnung: Lepidoptera, Coleoptera, Homoptera, Heteroptera, Diptera, Thysanoptera, Orthoptera, Blattaria, Anoplura, Siphonaptera, Mallophaga, Thysanura, Isoptera, Psocoptera und Hymenoptera eingesetzt werden.

Die Verbindung ist weiterhin geeignet zur Bekämpfung von Vertretern der Ordnung Acarina, z.B. der Familien Loxididae, Argasidae, Tetranychidae und Dermanyssidae. Die Verbindung kann mit Erfolg zur Bekämpfung von phy-

topathogenen Milben, z.B. der Familie Tetranychidae und Phytoptipalpidae (Spinnmilben), Tarsonemidae (Fadenfussmilben) und Eriophydiae (Gallmilben), eingesetzt werden.

Neben ihrer Wirkung gegenüber Mücken und Fliegen, wie z.B. Aedes aegypti und Musca domestica, kann die Verbindung auch zur Bekämpfung von pflanzenschädigenden Frassinsekten in Zier- und Nutzpflanzungen, insbesondere in Baumwollkulturen (z.B. gegen Spodoptera littoralis und Heliothis virescens), sowie in Getreide-, Obst- und Gemüsekulturen (z.B. gegen Laspeyresia pomonella, Leptinotarsa decemlineata und Epilachna varivestis) eingesetzt werden. Die Verbindung zeichnet sich auch durch eine gute Wirkung gegen larvale Entwicklungsstadien und Nymphen, insbesondere von fressenden Schadinsekten, aus.

Die Verbindung kann ferner zur Bekämpfung von ektoparasitären Insekten und Acariden an Haus- und Nutztieren verwendet werden, z.B. durch Tier-, Stall- und Weidebehandlung.

Herstellungsbeispiele

1. Stufe - **die Halogenierung:**

1a) 5,6-Dichlor-2,2-difluor-benzo(1.3)dioxol

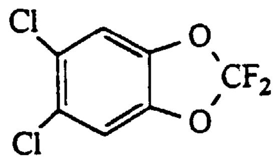

158 g (1 mol) 2,2-Difluorbenzo[1.3]dioxol werden mit 1,5 g Eisen-Pulver versetzt und unter Eiskühlung mit elementarem Chlor chloriert, bis das Dichlorprodukt entstanden ist (Gaschromatophie-Kontrolle ca. 8h). Durch Destillation erhält man 219 g (96 % der Theorie) an 5,6-Dichlor-2,2-difluor-benzo (1.3)dioxol mit Siedepunkt$_{18}$: 74 bis 78°C.

1b) 5,6-Dibrom-2,2-difluor-benzo(1.3)dioxol

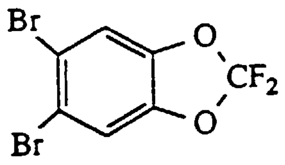

31,6 g (0,2 mol) 2,2-Difluorbenzo[1,3]dioxol ]dioxol werden mit 0,5 g Eisen-Pulver versetzt. Dann tropft man unter Eiskühlung bei 25°C Innentemperatur unter gutem Rühren innerhalb von 2 h 64 g (0,4 mol) Brom zu. Nach einstündigem Nachrühren bei 30°C gibt man auf Wasser, extrahiert mit Methylenchlorid, trocknet über Natriumsulfat und destilliert im Vakuum. Man erhält 36 g (57 % der Theorie) an 5,6-Dibrom-2,2-difluor-benzo (1.3)dioxol mit einem Siedepunkt$_{18}$: 108 bis 115°C.

2. Stufe - **die Nitrierung:**

2a) 5,6-Dichlor-4-nitro-2,2-difluor-benzo(1.3)dioxol

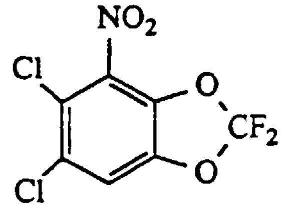

Zu 219 g (0,96 mol) 5,6-Dichlor-2,2-difluorbenzo[1.3]dioxol tropft man bei 5 bis 10°C (Innentemperatur) unter gutem

Rühren ein vorgekühltes Gemisch aus 140 ml 100 %iger Salpetersäure und 200 ml 100 %iger Schwefelsäure. Dann wird 6 h bei Raumtemperatur nachgerührt. Man gibt das Reaktionsgemisch auf Eiswasser extrahiert mit Ether, trocknet und engt ein. Man erhält 233 g (89 % der Theorie) 5,6-Dichlor-4-nitro-2,2-difluor-benzo(1.3)dioxol mit einem Schmelzpunkt von 29-30°C.

2b) 6,7-Dibrom-5-nitro-2,2,3,3-tetrafluor-benzo(1.4)dioxan

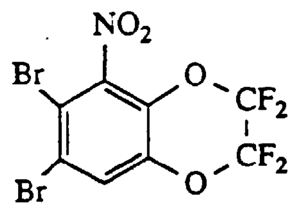

Zu 73 g (0,2 mol) 6,7-Dibrom-2,2,3,3-tetrafluor-benzo(1.4)dioxan tropft man bei 5°C langsam ein vorgekühltes Gemisch aus 19 ml Salpetersäure und 26 ml konzentrierter Schwefelsäure. Anschließend rührt man 4 Stunden bei 5°C nach, gibt auf Eis, extrahiert gründlich mit Ether, wäscht mit Wasser, Natriumhydrogencarbonat-Lösung (bis neutral), trocknet über Magnesiumsulfat und zieht den Ether ab. Man isoliert 72 g (88 % der Theorie) an 6,7-Dibrom-5-nitro-2,2,3,3-tetrafluor-benzo(1.4)dioxan mit dem Schmelzpunkt 91 bis 92°C.

3. **Reduktion der $NO_2$-Gruppe und selektive Dehalogenierung**

3a) 4-Amino-2,2-difluor-benzo(1.3)dioxol

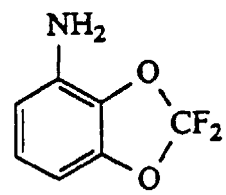

68 g (250 mmol) 5,6-Dichlor-4-nitro-2,2-difluor-benzo(1.3)dioxol, 69 g (500 mmol) Kaliumcarbonat und 5 g Palladium-Kohle (10 %ig) werden in 500 ml Methanol in einem Autoklaven 20 Stunden bei 50°C und einem Wasserstoff-Druck von 90 bar gerührt. Man filtriert die Reaktionsmischung. versetzt das Filtrat mit 500 ml Wasser, extrahiert gründlich mit Ether, trocknet über Natriumsulfat und rotiert den Ether ab. Durch Destillation erhält man 35 g (81 % der Theorie) 4-Amino-2,2-difluor-benzo(1.3)dioxol mit einem Siedepunkt von 84 bis 86°C/16 mm und einem Brechungsindex von $n_D^{21}$: 1.4995.

3b) 5-Amino-2,2,3,3-tetrafluor-benzo(1.4)dioxan

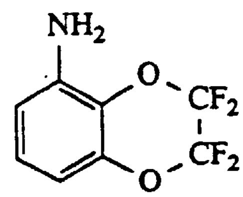

62 g (150 mmol) 6,7-Dibrom-5-nitro-2,2,3,3-tetrafluor-benzo(1.4)dioxan, 41 g (300 mmol) Kaliumcarbonat und 5 g Palladium-Kohle (10 %ig) werden in 500 ml Methanol in einem Autoklaven 20 Stunden bei 50°C und einem Wasserstoff-Druck von 90 bar gerührt Man filtriert die Reaktionsmischung, versetzt das Filtrat mit 500 ml Wasser, extrahiert gründlich mit Ether, trocknet über Natriumsulfat und engt ein. Eine Vakuum-Destillation liefert 26 g (78 % der Theorie) 5-Amino-2,2,3,3-tetrafluor-benzo(1.4)dioxan mit einem Siedepunkt von 99 bis 101°C/16 mm.

## Patentansprüche

1. Verfahren zur Herstellung von fluorsubstituierten Amino-benzodioxolen und -benzodioxanen der Formel

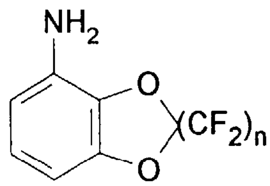

(I)

in welcher

n für eine ganze Zahl 1 oder 2 steht,

dadurch gekennzeichnet, daß man fluorsubstituierte Benzodioxole oder Benzodioxane der Formel

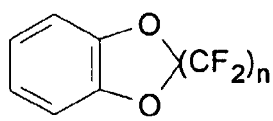

(II)

in welcher

n die oben angegebene Bedeutung hat,

in einer ersten Stufe mit elementarem Halogen in Gegenwart von Halogenierungskatalysatoren und gegebenenfalls in Gegenwart von Lösungs- oder Verdünnungsmitteln umsetzt und
in einer zweiten Stufe die so erhältichen halogensubstituierten Benzodioxole und Benzodioxane der Formel

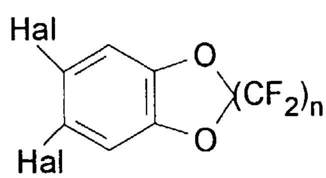

(III)

in welcher

n die obengenannte Bedeutung hat und

Hal für Halogen steht,

mit Nitriersäure ohne zusätzliche Lösungsmittel umsetzt und
in einer dritten Stufe die so erhältlichen Nitroverbindungen der Formel

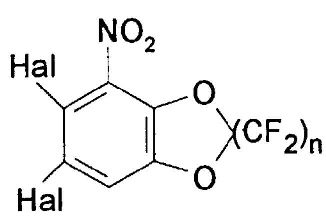

(IV)

in welcher
Hal und n die angegebenen Bedeutungen haben,
in Gegenwart von Palladiumkatalysatoren in Gegenwart eines Lösungsmittels und gegebenenfalls in Gegenwart einer Base mit Wasserstoff hydriert.

**2.** Verfahren nach Anspruch 1, dadurch gekennzeichnet, daß man in erster Stufe im Temperaturbereich zwischen -80 °C und 160 °C, in zweiter Stufe im Temperaturbereich zwischen -50 °C und 160 °C und in dritter Stufe im Temperaturbereich zwischen 20 °C und 180 °C arbeitet.

**3.** Verfahren nach Anspruch 1, dadurch gekennzeichnet, daß man in erster Stufe im Temperaturbereich zwischen -30 °C und 80 °C, in zweiter Stufe im Temperaturbereich zwischen -20 °C und 80 °C und in dritter Stufe im Temperaturbereich zwischen 30 °C und 140 °C arbeitet.

**4.** Verfahren nach Anspruch 1, dadurch gekennzeichnet, daß in Stufe 1 als Halogenierkatalysatoren Eisen oder Eisenverbindungen verwendet werden.

**5.** Verfahren nach Anspruch 1, dadurch gekennzeichnet, daß bei der katalytischen Hydrierung der Wasserstoffdruck im Bereich von 1 bis 200 bar liegt.

**6.** Fluorsubstituierte Nitro-benzodixole und benzodioxane der Formel

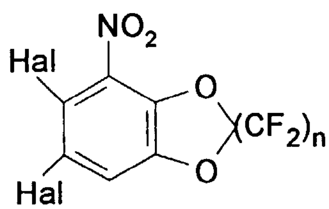

(IV)

in welcher

Hal für Halogen und

n für eine ganze Zahl 1 oder 2 stehen.

**7.** Halogensubstituierte Benzodioxole und Benzdioxane der Formel

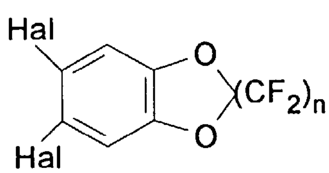

(III)

in welcher

Hal für Halogen und

n für eine ganze Zahl 1 oder 2 stehen.

**Claims**

**1.** Process for the preparation of fluorine-substituted amino-benzodioxoles and -benzodioxanes of the formula

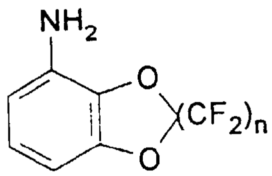

(I)

in which

n represents an integer 1 or 2,

characterised in that fluorine-substituted benzodioxoles or benzodioxanes of the formula

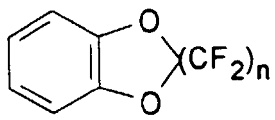

(II)

in which

n has the abovementioned meaning,

are reacted in a first step with elemental halogen in the presence of halogenation catalysts and if appropriate in the presence of solvents or diluents, and
in a second step the halogen-substituted benzodioxoles and benzodioxanes thus obtainable of the formula

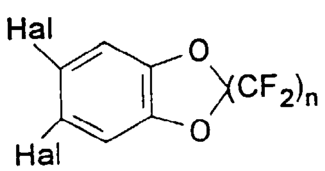

(III)

in which

n has the abovementioned meaning and

Hal represents halogen,

are reacted with nitrating acid without additional solvents, and
in a third step the nitro compounds thus obtainable of the formula

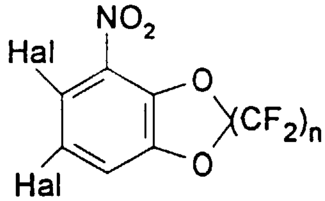

(IV)

in which
Hal and n have the meanings indicated,
are hydrogenated with hydrogen in the presence of palladium catalysts in the presence of a solvent and if appropriate in the presence of a base.

**2.** Process according to Claim 1, characterised in that the reaction is carried out in the first step in the temperature range between -80°C and 160°C, in the second step in the temperature range between -50°C and 160°C and in the third step in the temperature range between 20°C and 180°C.

**3.** Process according to Claim 1, characterised in that the reaction is carried out in the first step in the temperature range between -30°C and 80°C, in the second step in the temperature range between -20°C and 80°C and in the third step in the temperature range between 30°C and 140°C.

**4.** Process according to Claim 1, characterised in that iron or iron compounds are used in step 1 as halogenation catalysts.

**5.** Process according to Claim 1, characterised in that the hydrogen pressure during the catalytic hydrogenation is in the range from 1 to 200 bar.

**6.** Fluorine-substituted nitro-benzodioxoles and -benzodioxanes of the formula

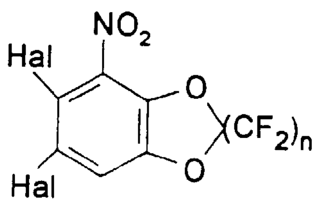

(IV)

in which

Hal represents halogen and

n represents an integer 1 or 2.

**7.** Halogen-substituted benzodioxoles and benzodioxanes of the formula

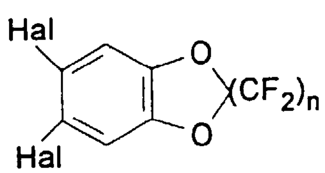

(III)

in which

Hal represents halogen and

n represents an integer 1 or 2.

**Revendications**

**1.** Procédé pour la préparation d'amino-benzodioxols et -benzodioxannes fluoro-substitués répondant à la formule

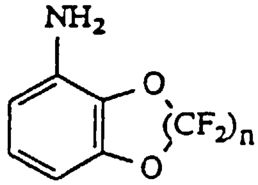

(I)

dans laquelle

n représente le nombre entier 1 ou 2,

caractérisé en ce qu'on fait réagir des benzodioxols ou des benzodioxannes fluoro-substitués répondant à la formule

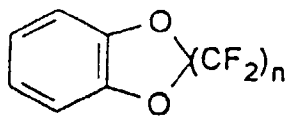

(II)

dans laquelle

n a la signification indiquée ci-dessus,

dans une première étape, avec un halogène élémentaire en présence de catalyseurs d'halogénation et éventuellement en présence de solvants ou de diluants, et
dans une deuxième étape, on fait réagir les benzodioxols et les benzodioxannes halogéno-substitués ainsi obtenus répondant à la formule

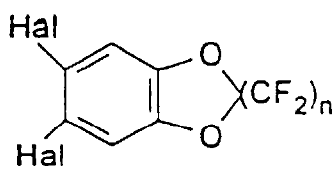

(III)

dans laquelle

n a la signification indiquée ci-dessus et

Hal représente un atome d'halogène,

avec un mélange sulfonitrique sans solvants supplémentaires, et
dans une troisième étape, on soumet à une hydrogénation avec de l'hydrogène les composés nitro ainsi obtenus répondant à la formule

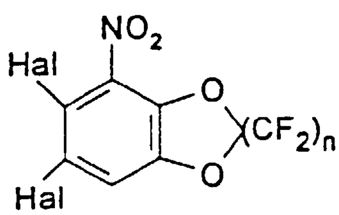

(IV)

dans laquelle
Hal et n ont les significations indiquées ci-dessus,
en présence de catalyseurs de palladium, en présence d'un solvant et éventuellement en présence d'une base.

**2.** Procédé selon la revendication 1, caractérisé en ce qu'on travaille dans la première étape dans le domaine de température entre -80°C et 160°C, dans la deuxième étape dans le domaine de température entre -50°C et 160°C, et dans la troisième étape dans le domaine de température entre 20°C et 180°C.

**3.** Procédé selon la revendication 1, caractérisé en ce qu'on travaille dans la première étape dans le domaine de température entre -30°C et 80°C, dans la deuxième étape dans le domaine de température entre -20°C et 80°C, et dans la troisième étape dans le domaine de température entre 30°C et 140°C.

**4.** Procédé selon la revendication 1, caractérisé en ce que, dans l'étape 1, on utilise comme catalyseurs d'halogénation, du fer ou des composés du fer.

**5.** Procédé selon la revendication 1, caractérisé en ce que, lors de l'hydrogénation catalytique, la pression de l'hydrogène se situe dans le domaine de 1 à 200 bar.

**6.** Nitro-benzodioxols et -benzodioxannes fluoro-substitués répondant à la formule

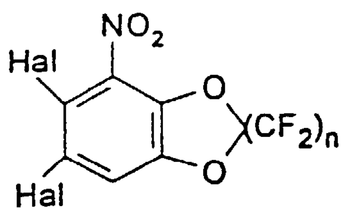

(IV)

dans laquelle

Hal représente un atome d'halogène et

n représente le nombre entier 1 ou 2.

**7.** Benzodioxols et benzodioxannes halogéno-substitués répondant à la formule

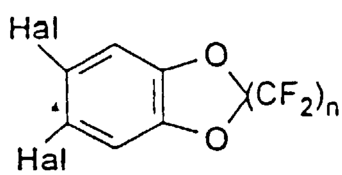

(III)

dans laquelle

Hal représente un atome d'halogène et

n représente le nombre entier 1 ou 2.